# EUROPÄISCHE PATENTSCHRIFT

(11) **EP 0 826 965 B1**
(45) Veröffentlichungstag und Bekanntmachung des Hinweises auf die Patenterteilung: **02.05.2003**
(21) Anmeldenummer: 97112829.3
(22) Anmeldetag: 25.07.1997
(51) Int. Cl.: G01N 33/86, C12Q 1/56, C07K 14/745

(54) **Verfahren zur Herstellung von Faktor V-Mangelplasma und ein so erhaltenes Mangelplasma**
Method for producing a factor V depleted plasma
Méthode de production d'un plasma appauvri en facteur V

(30) Priorität: 24.08.1996 DE 19634312
(43) Veröffentlichungstag der Anmeldung: 04.03.1998
(73) Patentinhaber: Dade Behring Marburg GmbH, 35001 Marburg (DE)
(72) Erfinder: Kraus, Michael, Dr., 35041 Marburg (DE); Aillaud, Erika, 35282 Rauschenberg (DE); Drescher, Heinz-Hermann, 35279 Neustadt (DE)

(56) Entgegenhaltungen:
- WO-A-94/17415
- WO-A-95/01571
- WO-A-96/15457
- KATZMANN ET AL: "Isolation of functional human coaggulation Factor V by using a hybridoma antibody." PROC.NATL.ACAD.SCI.USA, Bd. 78, Nr. 1, 1981, Seiten 161-166, XP002048309

## Beschreibung

Die Erfindung bezieht sich auf ein Verfahren zur Herstellung von Mangelplasmen, insbesondere eines Faktor V-Mangelplasmas aus einem Ausgangsplasma unter Verwendung von Antikörpern.

Unter Faktor V-Mangelplasma versteht man ein für Gerinnungsuntersuchungen geeignetes Plasma, das frei von Faktor V ist, jedoch alle anderen im Plasma normalerweise vorhandenen Gerinnungsfaktoren in im wesentlichen normaler Konzentration enthält.

Ein Faktor V-Mangelplasma ist geeignet, um in Faktor V-haltigen Flüssigkeiten, beispielsweise Blut oder Plasma, den Gehalt von aktivierbarem oder inaktivierbaren Faktor V in Kombination mit geeigneten Gerinnungstesten zu bestimmen.

Das Prinzip einer solchen Bestimmung besteht darin, die zu bestimmende Probe mit einem Überschuß an Faktor V-Mangelplasma zu mischen und einen Gerinnungstest bekannter Art durchzuführen. Das Ergebnis dieses Gerinnungstests hängt dann nur von dem in der zu untersuchenden Blut- oder Plasmaprobe vorhandenen Gehalt an Faktor V ab, da alle übrigen Gerinnungsfaktoren im Überschuß vorhanden und somit für die Reaktion nicht geschwindigkeitsbestimmend sind.

Für die Bestimmung von aktivierbarem Faktor V wird beispielsweise von der Mischung der Probe eine Thromboplastinzeit (TPZ) bestimmt. Hierbei wird die mit dem Mangelplasma gemischte Patientenprobe mit einem Gerinnungsaktivator, bei der TPZ handelt es sich um Thromboplastin, Phospholipiden und Calciumchlorid versetzt und die Gerinnungszeit bestimmt. In Abwesenheit von Faktor V ist die Gerinnung des Ansatzes deutlich verzögert, in Anwesenheit von Faktor V ist die Gerinnungszeit entsprechend der vorhandenen Faktor V Aktivität verkürzt. Dieses Verfahren ist beispielsweise von H. Stormorken (The preparation of proaccelerin deficient (parahemophilia) plasma for the assay of proaccelerin. Scand J Clin Lab Invest 9: 273, 1957) beschrieben worden.

Für die Bestimmung von inaktivierbarem Faktor V wird beispielsweise von der Mischung der Probe eine aktivierte partielle Thromboplastinzeit (APTT) in Gegenwart von aktiviertem Protein C durchgeführt (siehe beispielsweise: Kraus, M., Zander, N. and Fickenscher, K.: Coagulation assay with improved specificity to factor V mutants insensitive to activated protein C. Thrombosis Research, 80: 255-264, 1995; und EP 0 711 838). In Anwesenheit eines normal inaktivierbaren Faktor V ist die Gerinnungszeit in Gegenwart von aktiviertem Protein C gegenüber der APTT verlängert. Ist Faktor V aber nicht durch aktiviertes Protein C inaktivierbar, so ist die Verlängerung weniger stark ausgeprägt.

Die Empfindlichkeit insbesondere der Bestimmung von aktivierbarem Faktor V hängt entscheidend von der Qualität des verwendeten Mangelplasmas im Hinblick auf den Restgehalt an Faktor V im Mangelplasma ab, da die Bestimmung mit einem Überschuß an Mangelplasma durchgeführt wird und somit bereits ein geringer Anteil an Faktor V, beispielsweise 1% Restaktivität, nicht mehr tolerierbar ist.

Bei der Bestimmung von inaktivierbarem Faktor V, es handelt sich dabei um die Detektion eines auch als Faktor V-Leiden bezeichneten genetischen Defektes, sind daher diskrete Werte zu erwarten. So beträgt bei einem heterozygoten Träger des inaktivierbaren Faktor V die Verlängerung der Gerinnungszeit ca. 50%, bei einem homozygoten Träger nahe 0%. Hingegen zielt die Bestimmung von aktivierbarem Faktor V vor allem auf die Detektion erworbener Defizite, die etwa im Verlauf von Operationen oder bei Leberschäden auftreten, so daß für aktivierbaren Faktor V Werte im gesamten Konzentrationsbereich erwartet werden können. Für die Diagnose besonders wichtig ist die Erfassung von sehr niedrigen Konzentrationen an aktivierbarem Faktor V im Bereich etwa zwischen 1 und 10%. Das Risiko von Patienten mit einem Faktor V-Mangel unstillbare, innere Blutungen zu erleiden, steigt unterhalb von 10% Faktor V-Aktivität sehr stark an. Daher ist in diesem Bereich eine besonders exakte Bestimmung erforderlich, um über eine Therapie, z.B. mit Frischplasma, zu entscheiden. Wenn nun die Restaktivität des Faktor V in einem im Überschuß zugeführten Mangelplasma im Bereich von etwa 1 bis 5% liegt, wird der Gerinnungstest unempfindlich, d.h. die Bezugskurve zu flach, mit der Folge, daß in dem kritischen, niedrigen Konzentrationsbereich zwischen etwa 1 und 10% keine verläßlichen Messungen durchgeführt werden können.

Ein praktikables Mangelplasma sollte daher eine Faktor V Aktivität aufweisen, die deutlich unter 1% der Normalaktivität liegt.

Ein Mangelplasma einer Qualität, wie sie gemäß den obigen Schilderungen dringend erforderlich ist, kann das Plasma von Patienten sein, die selbst eine Faktor V-Aktivität aufweisen, die deutlich unter 1% liegt. Ein derartiges Mangelplasma ist naturgemäß selten, liegt nicht in der für Routinezwecke ausreichenden Menge vor und die Blutspende von Patienten, die an dieser schweren Form der Hämophilie leiden, ist mit ethischen Problemen behaftet.

Es ist daher erforderlich, andere Wege der Herstellung eines geeigneten Mangelplasmas zu suchen. Es bietet sich dabei an, auf physikalischchemischem Weg die Zerstörung des Faktor V vorzunehmen, wobei z.B. durch Zusatz von EDTA (Ethylendinitrilotetraessigsäure) und Erhitzung Faktor V denaturiert und dadurch inaktiviert wird. Ein Beispiel für dieses Vorgehen ist das Verfahren von Janssen et al. (Janssen, C.L., Wijngaards, G. and van der Meer, J.: Conditions for stabilization and determination of activated factor V. Thrombosis Research 5: 315-325, 1974). Dieses Mangelplasma ist jedoch für die Bestimmung des inaktivierbaren Faktor V nicht geeignet, da Faktor VIII ebenfalls durch den Zusatz von EDTA zerstört wird (siehe EP 0 711 838).

Ein anderes, bekanntes Verfahren besteht darin, Faktoren immunadsorptiv mittels Antikörper zu entfernen. Spezifische Antikörper werden an ein unlösliches Trägermaterial gebunden und das Plasma mit diesem Trägermaterial, das den spezifischen Antikörper gebunden hat, kontaktiert, so daß der jeweilige Faktor aus dem Plasma entfernt wird, wodurch ein Mangelplasma hergestellt werden kann. Im Gegensatz zu oben beschriebenen chemischen Verfahren werden andere Faktoren, insbesondere der für die Bestimmung des inaktivierbaren Faktor V wichtigen Faktor VIII, nicht zerstört. Daher können nur derart hergestellte Mangelplasmen für die Bestimmung von inaktivierbaren und aktivierbaren Faktor V verwendet werden (siehe EP 0 711 838; Kraus et al. 1995).

Bisher nach dem immunadsorptiven Verfahren hergestellte Faktor V-Mangelplasmen sind jedoch in ihrer Empfindlichkeit hinsichtlich aktivierbarem Faktor V dem chemischen Verfahren deutlich unterlegen. Dies ist exemplarisch in Beispiel 1 gezeigt, in dem je eine Referenzkurve für die Bestimmung von aktivierbarem Faktor sowohl mit einem chemisch als auch mit einem immunadsorptiv hergestellten Faktor V-Mangelplasma erstellt wurde. Die Spreizung der Referenzkurve im kritischen Bereich zwischen einem Faktor V-Gehalt von 1% und 10%, beträgt für das nach dem Stand der Technik immunadsorptiv gereinigte Plasma nur ca. 40% derjenigen Spreizung, die mit dem chemisch hergestellten Plasma erhalten wurde.

In WO9417415 wird die Herstellung eines Mangelplasmas beschrieben, bei dem Faktor V durch monoklonale oder polyklonale Antikörper entfernt wird.

In Katzmann et al. (1981), Proc. Natl. Acad. Sci. USA, Vol. 78, No. 1, S. 162-166 wird ein hochaffiner monoklonaler Antikörper beschrieben der zum Entfernen von Faktor V aus Lösungen geeignet ist.

Beschrieben worden ist auch schon die Verwendung einer Kombination von Antikörpern gegen zwei verschiedene Antigene, wie beispielsweise in EP 0 281 089 zur Herstellung eines Faktor VIII-Mangelplasmas. Da Faktor VIII:C an von Willebrand-Faktor gebunden im Plasma vorliegt, wurde eine entsprechend hohe Qualität, d.h. niedrige Konzentrationen an Faktor VIII:C erst mit einer

Kombination einer immunadsorptiven Reinigung gegen von Willebrand-Faktor und anschließend gegen Faktor VIII:C erzielt. In diesem Verfahren ist aber die Reihenfolge der Reinigungsschritte und eine hohe Spezifität der verwendeten Antikörper wichtig. Im Gegensatz dazu ist beim erfindungsgemäßen Verfahren die Reihenfolge der Reinigungsschritte unerheblich und es werden zweimal Antikörper gegen das gleiche Antigen benutzt.

Der Erfindung lag daher die Aufgabe zugrunde, ein Verfahren zur Herstellung eines Faktor V-Mangelplasmas zu entwickeln, das weniger als 1% des Normalwertes an Faktor V enthält und das eine hohe Empfindlichkeit bei der Bestimmung von aktivierbarem Faktor V vergleichbar zu bisherigen chemischen Verfahren aufweist, als auch die Bestimmung von inaktivierbaren Faktor V erlaubt.

Bei den immunadsorptiven Verfahren nach dem Stand der Technik zur Herstellung von Mangelplasmen werden entweder poly- oder monoklonale Antikörper verwendet. Überraschenderweise wurde nun gefunden, daß bei Verwendung einer Kombination von poly- und monoklonalen Antikörpern ein Faktor V-Mangelplasma in einer Qualität hergestellt werden kann, die mindestens vergleichbar, hier sogar besser als die nach dem chemischen Verfahren erzielte ist. Diese Qualität war nicht mit einer Kombination mehrerer monoklonaler Antikörper noch mit polyklonalen Antikörpern allein sondern überraschenderweise nur in Kombination beider Adsorptionsschritte zu erzielen, wobei die Reihenfolge dieser Schritte keine Rolle spielt.

Zweckmäßigerweise wird das Verfahren durchgeführt, indem die poly- oder monoklonalen Antikörper gegen Faktor V an unlösliche Trägermaterialien gebunden sind, die mit dem zu behandelnden Ausgangsplasma in Kontakt gebracht werden.

Die polyklonalen und die monoklonalen Antikörper können dabei in getrennten Fraktionen eingesetzt werden oder auch in einem Gemisch. Die Immunadsortion kann nach den hierfür ansich bekannten Verfahren z.B. also sowohl batchweise, als auch in Säulen erfolgen, wobei auch hier entweder getrennte Säulen für jede Antikörperart verwendet werden können oder ein Gemisch aus beiden Antikörperarten in eine Säule eingebracht werden kann.

Bei den unlöslichen Trägermaterialien handelt es sich bevorzugt um handelsübliche und dem Fachmann an sich bekannte Materialien.

Die verwendeten Antikörper sind zum einen polyklonal, zum anderen monoklonale Antikörper gegen humanen Faktor V, die im Handel von verschiedenen Herstellern angeboten werden.

Im erfindungsgemäßen Verfahren können nach der immunadsorptiven Entfernung von Faktor V andere Faktoren durch Zugabe von Faktorenkonzentraten nachträglich zugeführt werden. Hierzu werden handelsübliche Präparate verwendet, die frei von Faktor V sind, damit die Qualität des Mangelplasmas nachträglich nicht wieder vermindert wird.

Bevorzugterweise wird das Mangelplasma mit gereinigtem Faktor VIII oder Faktor VIII/von Willebrand-Faktor Gemisch und/oder Faktor IX auf 0,5 bis 2, besonders bevorzugt auf Konzentrationen von 0,8 bis 1,2 E/ml aufgefüllt.

Zur Neutralisierung von Heparin können weiterhin bekannte Neutralisatoren wie Protaminsulfat oder Hexadimethrin-Bromid (Handelsname: Polybren) in Konzentrationen von 1 bis 20 mg/l, besonders bevorzugt in Konzentrationen von 3 bis 10 mg/l zugegeben werden.

Mit dem beschriebenen Verfahren läßt sich ein Faktor V-Mangelplasma herstellen, daß weniger als 1% des Normalwertes an Faktor V enthält und mit dem eine Empfindlichkeit bei der Bestimmung von aktivierbarem Faktor V vergleichbar mit chemisch hergestellten Faktor V-Mangelplasmen erzielt wird, während die Bestimmung von inaktivierbarem Faktor V ebenfalls möglich ist.

Bevorzugterweise beträgt die Spreizung der Referenzkurve in dem kritischen Bereich von einem Faktor V-Gehalt von 1 bis 10% mindestens 50%, ganz bevorzugterweise mindestens 70% der Spreizung bei einem chemisch hergestellten, d.h. chemisch inaktiviertem, Mangelplasma, besonders bevorzugterweise beträgt sie mindestens 90% oder ist besser als bei einem chemisch hergestellten, d.h. chemisch inaktiviertem, Mangelplasma.

Das beschriebene Verfahren kann auch zur Herstellung von Mangelplasmen für andere Faktoren verwendet werden um eine erhöhte Empfindlichkeit im Bereich 1 bis 10% des gesuchten Faktors in Gerinnungstesten zu erzielen. Es können dabei auch Mangelplasmen erzeugt werden, die Mangelplasmen in Bezug auf mehr als einen Faktor sind.

Die Erfindung wird durch die nachfolgenden Beispiele näher erläutert. Falls nicht anders ausgeführt wurden die Reagenzien der Fa. Behringwerke AG, Marburg, Deutschland, verwendet.

### Vergleichsbeispiel

**Vergleich der Empfindlichkeit von nach dem Stand der Technik chemisch und immunadsorptiv hergestelltem Faktor V-Mangelplasma hinsichtlich der Bestimmung von aktivierbarem Faktor V.**

Mit einem Plasma mit kalibrierten Faktor V-Gehalt (Standardhumanplasma, Prod. Nr. ORKL; FV-Gehalt: 100%) wurde eine Bezugskurve mit folgenden Faktor V-Mangelplasmen erstellt: chemisch hergestelltes Faktor V-Mangelplasma (Faktor V-Mangelplasma, Prod. Nr. ORSM) bzw. immunadsorptiv hergestelltes Faktor V-Mangelplasma (Faktor V-Mangelplasma, Prod. Nr. 291020, Fa. Baxter, Unterschleissheim, Deutschland). Hierzu wurde das Standardplasma zunächst auf Konzentrationen von 100, 50, 10 und 1% mit Imidazol-Pufferlösung (Prod. Nr. OQAA) und anschließend nochmals mit Imidazol-Pufferlösung 1:20 weiter verdünnt. Als 0%-Wert wurde Imidazol-Pufferlösung selbst verwendet. 100 µl des jeweiligen Faktor V-Mangelplasmas und 100 µl Probenverdünnung wurden in einem Koagulometer (Fa. Amelung, Lemgo, Deutschland) gemischt und 60 Sekunden bei +37°C erwärmt. Durch Zugabe von 200 µl eines auf +37°C vortemperierten Thromboplastin-Reagenzes (Thromborel S, Prod. Nr. OUHP) wurde die Gerinnung ausgelöst und die Gerinnungszeit bestimmt.

In Tabelle 1 sind die Gerinnungszeiten aufgeführt, die mit dem jeweiligen, handelsüblichen Faktor V-Mangelplasma erhalten wurden. Es ist ersichtlich, daß die Gesamtspreizung, insbesondere aber die Empfindlichkeit im, für die Bestimmung des aktivierbaren Faktor V so wichtigen Bereichs unterhalb von 10% Faktor V-Gehalt, mit dem immunadsorptiv hergestellten Faktor V-Mangelplasma um ca. 60% geringer ist gegenüber derjenigen des chemisch hergestellten Faktor V-Mangelplasmas.

**Tabelle 1:**

| | | |
|---|---|---|
| Gerinnungszeiten in Sekunden bei der Kalibration der Bestimmung an aktivierbarem Faktor V mit 2 kommerziellen Faktor V-Mangelplasmen hergestellt nach bisherigen Verfahren. | | |
| Weiterhin ist die Differenz der Gerinnungszeit zwischen einer Faktorenkonzentration von 10 bzw. 1% als Maß der Nachweisempfindlichkeit von Faktor V aufgeführt. | | |

| Faktor V-Konzentration [%] | chemisch hergestelltes FV-Mangelplasma | immunadsorptiv hergestelltes FV-Mangelplasma |
|---|---|---|
| 0 | 109,3 | 59,0 |
| 1 | 102,5 | 57,0 |
| 10 | 72,5 | 45,0 |
| 50 | 46,8 | 32,2 |
| 100 | 38,1 | 26,0 |
| Differenz 10%-1% | 30,0 | 12,0 |

### Beispiel 1

### Immunadsorptive Herstellung von Faktor V-Mangelplasma.

Für die erfindungsgemäße Herstellung von Faktor V-Mangelplasma wurden 10 mg der jeweiligen Antikörper an je 5 g Bromcyan-aktivierter Sepharose (Fa. Pharmacia, Uppsala, Schweden) kovalent immobilisiert. Die Kopplungsreaktion folgte einem beschriebenen Verfahren (Axen, R. et al., Nature, 214: 1302, 1967) gemäß den Angaben des Herstellers. Insgesamt wurden 7 verschiedene monoklonale Antikörper untersucht. Für die in den Beispielen beschriebenen Untersuchungen wurden die in Tabelle 2 aufgeführten Antikörper (Fa. Behringwerke AG) ausgewählt. Quantitative Unterschiede zu den in den Beispielen beschriebenen Ergebnissen gab es nicht.

Anschließend wurde das Immunadsorbens jeweils mit phosphatgepufferter Kochsalzlösung (PBS; 0,15 mol/L, pH 7,2) und Essigsäure (0,5 mol/l, pH 2,5) gewaschen. Vor der Verwendung wurde jedes Adsorbens in eine eigene Säule gepackt und mit dem 3-fachen Gelvolumen PBS äquilibriert. Über eine Säule oder eine Kette hintereinander geschalteter Säulen wurde Citratplasma aus Humanblut geschickt und das Eluat fraktioniert aufgefangen. Für die weiteren Analysen wurden nur Fraktionen verwendet, die maximal adsorbiert waren um eine Verfälschung der Ergebnisse durch eine etwaige unterschiedliche Adsorptionskapazität der Säule auszuschließen. Hierzu wurde in jeder Fraktion der Faktor V-Gehalt bestimmt. Nur Fraktionen mit der längsten Gerinnungszeit in der Faktor V-Bestimmung und einem Faktor V-Gehalt deutlich unter 1% wurden verwendet. Weiterhin wurden nur solche Fraktionen verwendet, die sich in ihrem Verhalten nicht von den ersten Fraktionen nach Erreichen der optimalen Qualität im jeweiligen Lauf unterschieden. Nach diesen Kriterien betrug beispielsweise die Kapazität einer Kombination von 750 mg MAk 95-240/05 und 360 mg PAK 6000 ml Humanplasma. Für die Untersuchungen in den folgenden Beispielen wurden aber nur ca. 2 ml benötigt.

**Tabelle 2**

| | |
|---|---|
| | |
| In den Beispielen verwendete Antikörper. | |

| Bezeichnung | Typ |
|---|---|
| PAK | polyklonale Antikörper vom Kaninchen |
| 95-240/05 | monoklonaler Antikörper |
| 95-275/033 | monoklonaler Antikörper |

### Beispiel 2

**Vergleich der Empfindlichkeit von mit verschiedenen, immunadsorptiven Verfahren hergestellten Faktor V-Mangelplasmen hinsichtlich der Bestimmung von aktivierbarem Faktor V.**

Die unter Beispiel 1 hergestellten Immunadsorbentien wurden allein und in verschiedenen Kombinationen zur Herstellung von Faktor V-Mangelplasma verwendet. Die Analyse der Empfindlichkeit zum Nachweis von aktivierbaren Faktor V wurde gemäß Beispiel 1 durchgeführt.

In Tabelle 3 sind die Gerinnungszeiten aufgeführt, die mit diesen verschiedenen Kombinationen erhalten wurden. Als Maß der Empfindlichkeit ist unter "Sens." die Differenz zwischen der Gerinnungszeit bei 10 und 1% Faktor V-Gehalt aufgeführt, die Gesamtspreizung der Referenzkurve zwischen 0 und 100% ist in der Zeile "Total" beschrieben. Es ist ersichtlich, daß es weder mit polyklonalen Antikörper allein, noch mit monoklonalen Antikörpern allein oder in Kombination mehrerer monoklonaler Antikörper gelang eine Qualität eines Faktor V-Mangelplasmas zu erzeugen, die dem chemisch erzeugten Produkt in Beispiel 1, Tabelle 1, entspricht. Einzig die Kombination des polyklonalen und eines monoklonalen Antikörpers ergab nicht nur vergleichbare, sondern sogar um ca. 10% verbesserte Qualität hinsichtlich der Empfindlichkeit bei etwa gleicher Gesamtspreizung der Referenzkurve. Die Reihenfolge der Adsorptionsschritte ist dabei unerheblich.

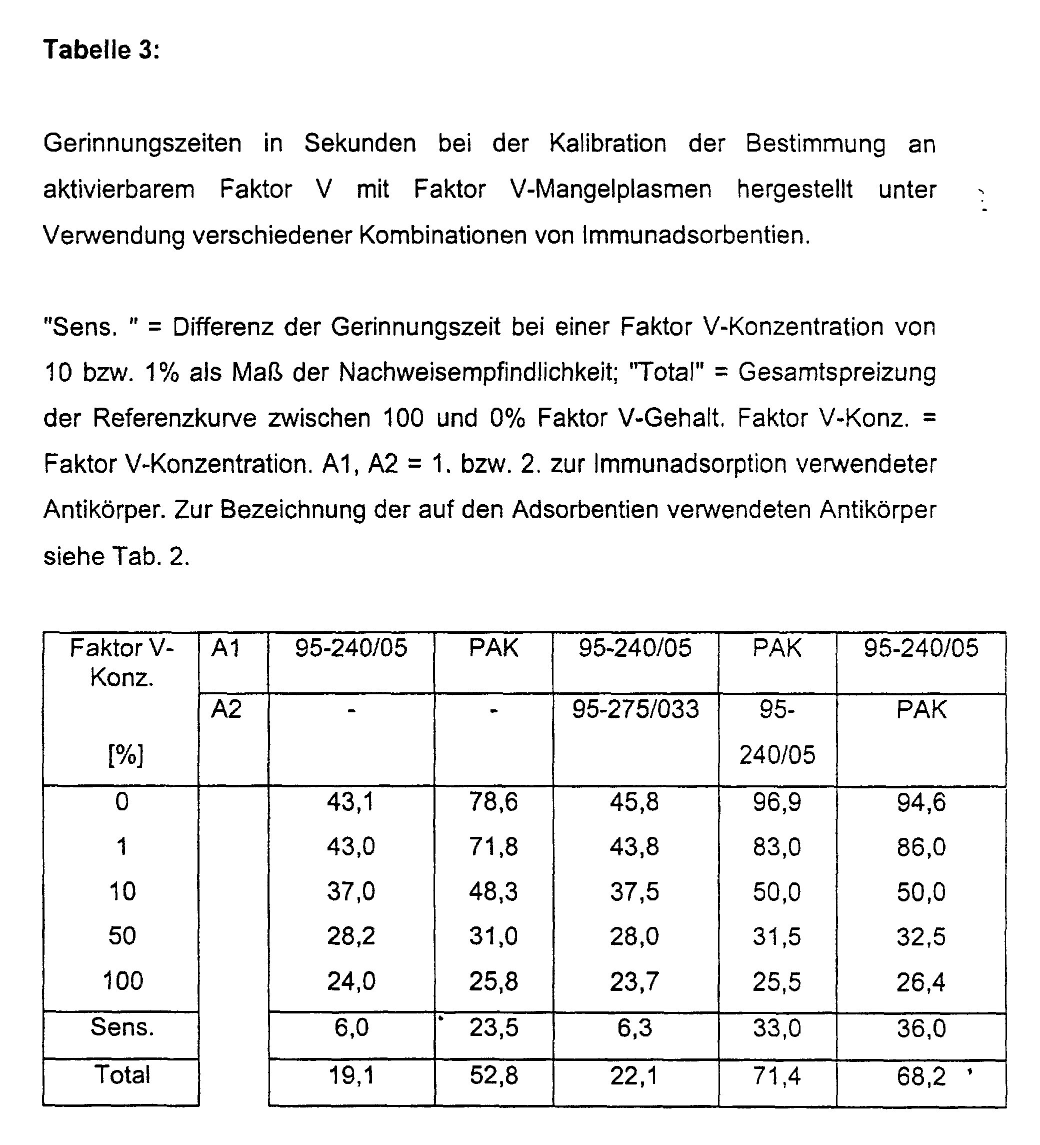

### Beispiel 3

### Eignung des erfindungsgemäß hergestellten Faktor V-Mangelplasmas zur Bestimmung von inaktivierbarem Faktor V (Faktor V-Leiden)

Ein nach der erfindungsgemäßen Kombination einer polyklonalen und einer monoklonalen Immunadsorption hergestelltes Faktor V-Mangelplasma wurde auf seine Eignung auf die Detektion eines inaktivierbaren Faktor V geprüft. Hierzu wurde die Gerinnungszeit eines normalen Plasmapools (Standardhumanplasma; Prod. Nr. ORKL, Fa. Behringwerke AG) sowie Citratplasma von einem Spender mit heterozygotem Faktor V-Leiden Defekt unter Verwendung von ProC APC (Prod. Nr. OQKF, Fa. Behringwerke AG) an einem Koagulometer (Behring Coagulation Timer, Fa. Behringwerke AG) bestimmt. Zur Durchführung wurden 10 µl Plasmaprobe mit 40 µl Faktor V-Mangelplasma gemischt und mit 50 µl eines Kontaktphasenaktivators (Pathromtin SL; Prod. Nr. OQGS) versetzt. Zur Bestimmung der APTT wurde nach 2-minütiger Inkubation bei +37°C die Gerinnung durch Zugabe von 50 µl 25 mmol/L Calciumchloridlösung gestartet, zur Bestimmung der APTT in Anwesenheit von aktiviertem Protein C (APCT) wurde nach 3-minütiger Inkubation bei +37°C die Gerinnung durch Zugabe des im Kit befindlichen APC-Reagenzes (enthält aktiviertes Protein C und Calciumchlorid) ausgelöst. Die Gerinnungszeiten wurden bestimmt und sind in Tabelle 4 aufgeführt.

Unter Verwendung des erfindungsgemäß hergestellten Faktor V-Mangelplasmas wird im Test auf Faktor V-Leiden mit einem Pool normaler Blutspender eine Verlängerung der APTT um 57,5 sec gefunden, bedingt durch die Inaktivierung des normalen Faktor V durch die Anwesenheit von aktivierten Protein C. Bei einem heterozygoten Faktor V Leiden-Träger hingegen ist die Hälfte der im Plasma vorkommenden Faktor V-Moleküle derart verändert, daß sie nur sehr langsam durch aktiviertes Protein C inaktiviert werden, während die prokoagulatorische Aktivität davon unberührt bleibt. Entsprechend ist daher unter Einwirkung von aktiviertem Protein C nur mit einer ca. 1/2-fachen Verlängerung der Gerinnungszeit im Vergleich zum Normalplasma Pool zu rechnen, wie dies mit nur 27,5 sec entsprechend erhalten wird.

Das erfindungsgemäße Verfahren ist somit geeignet ein Faktor V-Mangelplasma in ausreichender Qualität auch zur Bestimmung der Inaktivierbarkeit von Faktor V zu liefern.

**Tabelle 4:**

| | | |
|---|---|---|
| Prüfung der Eignung des im erfindungsgemäßen Verfahren hergestellten Faktor V-Mangelplasmas zur Detektion eines Faktor V-Leiden Defekts. | | |
| APTT = aktivierte, partielle Thromboplastin-Zeit; APCT = APTT in Anwesenheit von aktiviertem Protein C. Angaben der Gerinnungszeit in Sekunden. | | |

| Gerinnungstest | Faktor V-Leiden Defekt | Normalplasma Pool |
|---|---|---|
| APTT | 50,5 | 48,3 |
| APCT | 78,0 | 105,8 |
| Differenz APCT-APTT | 27,5 | 57,5 |

## Patentansprüche

1. Verfahren zur Herstellung eines Faktor V-Mangelplasmas, das weniger als 1% des Normalwertes an Faktor V enthält, aus einem Ausgangsplasma unter Verwendung von Antikörpern gegen Faktor V, **dadurch gekennzeichnet, daß** das Ausgangsplasma durch Immunadsorption mit monoklonalen und polyklonalen Antikörpern gegen Faktor V gereinigt wird.

2. Verfahren nach Anspruch 1, **dadurch gekennzeichnet, daß** das Ausgangsplasma in einem ersten Schritt mit polyklonalen Antikörpern und in einem zweiten Schritt mit monoklonalen Antikörpern behandelt wird.

3. Verfahren nach Anspruch 1, **dadurch gekennzeichnet, daß** das Ausgangsplasma in einem ersten Schritt mit monoklonalen Antikörpern und in einem zweiten Schritt mit polyklonalen Antikörpern behandelt wird.

4. Verfahren nach Anspruch 1, **dadurch gekennzeichnet, daß** das Ausgangsplasma mit einem Gemisch aus polyklonalen Antikörpern und monoklonalen Antikörpern behandelt wird.

5. Verfahren nach einem der Ansprüche 1 bis 4, **dadurch gekennzeichnet, daß** die genannten Antikörper an unlösliche, handelsübliche Trägermaterialien gebunden sind.

6. Verfahren nach einem der Ansprüche 1 bis 4, **dadurch gekennzeichnet, daß** als polyklonale Antikörper Antikörper gegen humanen Faktor V verwendet werden, die aus einer Warmblüterspezies, bevorzugterweise jedoch aus Kaninchen, Schaf, Huhn oder Ziege gewonnen wurden.

7. Verfahren nach einem der Ansprüche 1 bis 4, **dadurch gekennzeichnet, daß** als monoklonale Antikörper Antikörper gegen human Faktor V verwendet werden, wobei ein oder mehrere verschiedene Klone, bevorzugterweise aber nur Antikörper eines Klones eingesetzt werden.

8. Verfahren nach einem der Ansprüche 1 bis 4, **dadurch gekennzeichnet, daß** Faktor VIII:C, von Willebrand-Faktor oder von Willebrand-Faktor / Faktor VIII:C-Gemisch vor oder nach der Herstellung dem Faktor V-Mangelplasma zugesetzt werden, um im Faktor V-Mangelplasma Konzentrationen von 0,6 - 2, bevorzugterweise Konzentrationen von 0,8 - 1,2 E/ml zu erzielen.

9. Verfahren nach einem der Ansprüche 1 bis 4, **dadurch gekennzeichnet, daß** Faktor IX vor oder nach der Herstellung dem Faktor V-Mangelplasma zugesetzt werden, um im Faktor V-Mangelplasma Faktor IX Konzentrationen von 0,6 - 2, bevorzugterweise Konzentrationen von 0,8 - 1,2 E/ml zu erzielen.

10. Verfahren nach Anspruch 8 oder 9, **dadurch gekennzeichnet, daß** sowohl Faktor VIII:C, von Willebrand-Faktor oder von Willebrand-Faktor / Faktor VIII:C-Gemisch als auch Faktor IX zugegeben werden.

11. Verfahren nach einem der Ansprüche 1 bis 10, **dadurch gekennzeichnet, daß** Heparinneutralisatoren wie Protaminsulfat oder Hexadimethrin-Bromid in Konzentrationen von 1 bis 20 mg/l, bevorzugterweise in Konzentrationen von 3 bis 10 mg/l zugegeben werden.

12. Mangelplasma hergestellt nach einem der Ansprüche 1 bis 11.

13. Verwendung eines Mangelplasmas nach Anspruch 12 in einem diagnostischen Verfahren.

14. Verwendung eines Mangelplasmas nach Anspruch 12 in einem diagnostischen Verfahren zur Bestimmung von Faktor V-Leiden.

## Claims

1. A process for preparing a factor V-deficient plasma, which contains less than 1% of the normal value of factor V, from a starting plasma using antibodies against factor V, which comprises purifying the starting plasma by immunoadsorption with monoclonal and polyclonal antibodies against factor V.

2. The process as claimed in claim 1, wherein the starting plasma is treated with polyclonal antibodies in a first step and with monoclonal anti-bodies in a second step.

3. The process as claimed in claim 1, wherein the starting plasma is treated with monoclonal antibodies in a first step and with polyclonal antibodies in a second step.

4. The process as claimed in claim 1, wherein the, starting plasma is treated with a mixture of polyclonal antibodies and monoclonal antibodies.

5. The process as claimed in one of claims 1 to 4, wherein the said antibodies are bound to insoluble, commercially available support materials.

6. The process as claimed in one of claims 1 to 4, wherein antibodies against human factor V which were isolated from any homeothermic species, preferably, however, from rabbit, sheep, chicken or goat, are used as the polyclonal antibodies.

7. The process as claimed in one of claims 1 to 4, wherein antibodies against human factor V are used as the monoclonal antibodies, with one or more different clones, preferably, however, only antibodies of one clone, being employed.

8. The process as claimed in one of claims 1 to 4, wherein factor VIII:C, von Willebrand factor or a von Willebrand factor/factor VIII:C mixture is added to the factor V-deficient plasma before or after the preparation in order to achieve concentrations of 0.6 - 2, preferably concentrations of 0.8 - 1.2, U/ml in the factor V-deficient plasma.

9. The process as claimed in one of claims 1 to 4, wherein factor IX is added to the factor V-deficient plasma before or after the preparation in order to achieve factor IX concentrations of 0.6 - 2, preferably concentrations of 0.8 - 1.2, U/ml in the factor V-deficient plasma.

10. The process as claimed in claim 8 or 9, wherein both factor VIII:C, von Willebrand factor or a von Willebrand factor/factor VIII:C mixture and factor IX are added.

11. The process as claimed in one of claims 1 to 10, wherein heparin neutralizers such as protamine sulfate or hexadimethrine bromide are added at concentrations of from 1 to 20 mg/l, more preferably at concentrations of from 3 to 10 mg/l.

12. A deficient plasma which is prepared as claimed in one of claims 1 to 11.

13. The use of a deficient plasma as claimed in claim 12 in a diagnostic process.

14. The use of a deficient plasma as claimed in claim 12 in a diagnostic process for determining factor V disorder.

## Revendications

1. Procédé de production d'un plasma pauvre en facteur V, qui présente moins de 1 % de la teneur normale en facteur V, à partir d'un plasma de départ, avec utilisation d'anticorps contre le facteur V, **caractérisé en ce que** le plasma de départ est purifié par immunoadsorption avec des anticorps monoclonaux et des anticorps polyclonaux dirigés contre le facteur V.

2. Procédé selon la revendication 1, **caractérisé en ce que** le plasma de départ est traité dans une première étape par des anticorps polyclonaux et dans une seconde étape par des anticorps monoclonaux.

3. Procédé selon la revendication 1, **caractérisé en ce que** le plasma de départ est traité dans une première étape par des anticorps monoclonaux et dans une seconde étape par des anticorps polyclonaux.

4. Procédé selon la revendication 1, **caractérisé en ce que** le plasma de départ est traité par un mélange d'anticorps polyclonaux et d'anticorps monoclonaux.

5. Procédé selon l'une quelconque des revendications 1 à 4, **caractérisé en ce que** lesdits anticorps sont fixés sur des matériaux de support insolubles du commerce.

6. Procédé selon l'une quelconque des revendications 1 à 4, **caractérisé en ce qu'**on utilise comme anticorps polyclonaux des anticorps dirigés contre le facteur V humain, qui ont été obtenus à partir d'une espèce d'animal à sang chaud, mais de préférence à partir du lapin, du mouton, de la poule ou de la chèvre.

7. Procédé selon l'une quelconque des revendications 1 à 4, **caractérisé en ce qu'**on utilise comme anticorps monoclonaux des anticorps dirigés contre le facteur V humain, en utilisant un ou plusieurs clones différents, mais de préférence seulement des anticorps d'un clone.

8. Procédé selon l'une quelconque des revendications 1 à 4, **caractérisé en ce que**, avant ou après la production, on ajoute du facteur VIII:C, du facteur von Willebrand ou un mélange facteur VIII:C/facteur von Willebrand au plasma pauvre en facteur V, afin d'obtenir dans le plasma pauvre en facteur V des concentrations de 0,6-2, de préférence des concentrations de 0,8-1,2 U/ml.

9. Procédé selon l'une quelconque des revendications 1 à 4, **caractérisé en ce que**, avant ou après la production, on ajoute du facteur IX au plasma pauvre en facteur V, afin d'obtenir dans le plasma pauvre en facteur V des concentrations de facteur IX de 0,6-2, de préférence des concentrations de 0,8-1,2 U/ml.

10. Procédé selon la revendication 8 ou 9, **caractérisé en ce qu'**on ajoute aussi bien du facteur VIII:C, du facteur von Willebrand ou un mélange facteur VIII:C/facteur von Willebrand que du facteur IX.

11. Procédé selon l'une quelconque des revendications 1 à 10, **caractérisé en ce qu'**on ajoute des agents de neutralisation de l'héparine, tels que le sulfate de protamine ou le bromure d'hexadiméthrine, à des concentrations de 1 à 20 mg/l, de préférence à des concentrations de 3 à 10 mg/l.

12. Plasma appauvri, produit selon l'une quelconque des revendications 1 à 11.

13. Utilisation d'un plasma appauvri selon la revendication 12, dans un procédé de diagnostic.

14. Utilisation d'un plasma appauvri selon la revendication 12, dans un procédé de diagnostic pour la détermination de maladies en relation avec le facteur V.
